# EUROPEAN PATENT APPLICATION

(11) **EP 4 467 651 A1**
(43) Date of publication of application: **27.11.2024**
(21) Application number: 23742994.9
(22) Date of filing: 20.01.2023
(51) Int. Cl.: C12N 15/70, C12N 15/81, C12N 9/02, C12N 15/53, C12P 17/06, C12N 1/21, C12N 15/62, C12N 15/75, C12N 1/19, C07K 19/00, C12R 1/19

(54) **METHOD FOR REGULATING AND CONTROLLING HETEROLOGOUS SYNTHETIC FLAVONOID COMPOUND AND USE THEREOF**

(30) Priority: 20.01.2022 CN 202210066386
(71) Applicant: CAS Center for Excellence in Molecular Plant Sciences, Shanghai 200032 (CN)
(72) Inventor: WANG, Yong, Shanghai 200032 (CN); LI, Jianhua, Shanghai 200032 (CN); JI, Dongni, Shanghai 200032 (CN)
(74) Representative: EP&C
(86) International application number: PCT/CN2023/073308
(87) International publication number: WO 2023/138679

(57) **Abstract**

Provided is a method for synthesizing a flavonoid compound. The method comprises providing a recombinant prokaryotic cell, wherein, in the prokaryotic cell, the transmembrane protein rhodanese Ygap of *Escherichia coli* is up-regulated or a target gene or target gene combination selected from the following groups is down-regulated: pyrB, accC, accB, purC, glyA, tktA, fabB, leuD, leuC, glpC, folK and leuA. Also provided are a prokaryotic cell for synthesizing a flavonoid compound and the use thereof, and the use of a kit and a regulation and control reagent. The present disclosure achieves significant improvement in the yield of the flavonoid compound.

## Description

### FIELD OF THE INVENTION

The disclosure refers to the field of synthetic biology and medicine, in particular, the disclosure relates to method for regulating and controlling heterologous synthetic flavonoid compound and use thereof.

### BACKGROUND OF THE INVENTION

Baicalein and scutellarein are flavonoid compounds isolated from the traditional Chinese medicine, *Scutellaria baicalensis.* These two bioactive flavonoids accumulate in only small amounts in the roots of medicinal plants such as *Scutellaria baicalensis.* Baicalein and scutellarein are synthesized through flavonoid biosynthesis. Baicalein and scutellarein have significant physiological activities, including antioxidant, antitumor, antibacterial and cardioprotective effects. Recently, baicalein has been reported in vitro as an inhibitor of SARS-CoV-2 3Clpro, demonstrating the great potential of traditional Chinese medicine.

Currently, main sources of flavonoids are plant extraction and chemical synthesis. However, due to the use of toxic chemicals and extreme reaction conditions, plant extraction or chemical synthesis cannot provide a green route for large-scale production. Therefore, researches on microbial synthesis of flavonoids have been extensively carried out. Due to complex heterologous pathways introduced in plants, enzyme imbalances, and accumulation of intermediate metabolites, the titer of products usually remains at a low level. To address these issues, a multivariable modular approach is used for synthesizing flavonoids by regulating promoter strength and plasmid copy number. However, this approach is time-consuming and always requires extensive works. Previous studies have reported the synthesis of baicalein and scutellarein in engineered yeast and *E. coli,* but the yields of baicalein and scutellarein remain very low.

Therefore, there is an urgent need in this field to optimize microbial strains capable of efficiently synthesizing baicalein, scutellarein or similar compounds heterologously.

### SUMMARY OF THE INVENTION

The aim of the present disclosure is to provide a method for regulating and controlling heterologous synthetic flavonoid compound and use thereof.

In the first aspect, the present disclosure provides a method for synthesizing a flavonoid compound, wherein the method comprises:
(1) providing a recombinant prokaryotic cell, comprising exogenous genes encoding the following enzymes: phenylalanine ammonia lyase (PAL), 4-coumaryl-CoA ligase (4CL), chalcone synthase (CHS), chalcone isomerase (CHI) and flavone synthase I (FNSI); and, in the prokaryotic cell, the transmembrane protein rhodanese Ygap of *Escherichia coli* is up-regulated or a target gene or target gene combination selected from the following group is down-regulated: pyrB, accC, accB, purC, glyA, tktA, fabB, leuD, leuC, glpC, folk, leuA;
(2) using the formula (I) as a substrate to synthesize flavonoids with the prokaryotic cells, wherein the flavonoids are chrysin compounds (such as chrysin or apigenin); 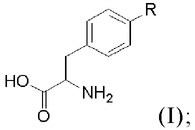 wherein, R comprises H or OH.

In one or more embodiments, in (1), the prokaryotic cell also comprises exogenous genes encoding the following enzymes: flavone 6-hydroxylase (F6H) and cytochrome P450 oxidoreductase (CPR); in (2), the flavonoid compound is baicalein compound (such as baicalein or scutellarein).

In one or more embodiments, the down-regulation of target genes comprises: down-regulating the activity of target genes, decreasing the effective time of target genes, down-regulating the expression or stability of target genes, and so on.

In one or more embodiments, the up-regulated transmembrane protein rhodanese Ygap of *Escherichia coli* comprises: introducing an exogenous gene encoding transmembrane protein rhodanese Ygap of *Escherichia coli* into the prokaryotic cell; preferably the exogenous gene encoding transmembrane protein rhodanese Ygap of *Escherichia coli* is introduced into the prokaryotic cell via an expression vector.

In one or more embodiments, the down-regulation of target gene comprises: knocking out or silencing the target gene in the cell, or inhibiting the activity of the target gene; preferably, knocking out or silencing the target gene in the cell comprises (but is not limited to): silencing the target gene with a specific interfering molecule, knocking out the target gene by gene editing with a CRISPR system, knocking out the target gene by homologous recombination, or mutating the target gene by loss-of-function mutation; preferably, the interfering molecule comprises sRNA.

In one or more embodiments, the interfering molecule is sRNA, with the sRNA sequence targeting glpC is as shown in SEQ ID NO: 2; the sRNA sequence targeting leuA is as shown in SEQ ID NO: 3; the sRNA sequence targeting leuC is as shown in SEQ ID NO: 4; the sRNA sequence targeting leuD is as shown in SEQ ID NO: 5; the sRNA sequence targeting folK is as shown in SEQ ID NO: 6; the sRNA sequence targeting tktA is as shown in SEQ ID NO: 7; the sRNA sequence targeting fabB is as shown in SEQ ID NO: 8; the sRNA sequence targeting accC is as shown in SEQ ID NO: 9; the sRNA sequence targeting accB is as shown in SEQ ID NO: 10; the sRNA sequence targeting purC is as shown in SEQ ID NO: 11; the sRNA sequence targeting pyrB is as shown in SEQ ID NO: 12; or the sRNA sequence targeting glyA is as shown in SEQ ID NO: 13.

In one or more embodiments, agents for down-regulating target genes are called down-regulating agents or down-regulators (including inhibitors, blockers, antagonists, etc.).

In one or more embodiments, the phenylalanine ammonia lyase (PAL) and 4-coumaryl-CoA ligase (4CL) are configured to form a complex (complex reactor).

In one or more embodiments, the phenylalanine ammonia lyase and the 4-coumaryl-CoA ligase are approached to obtain a complex through the binding of a domain for protein-protein interaction and the binding with its ligand, or the phenylalanine ammonia lyase and the 4-coumaryl-CoA ligase are linked directly or using a linker to obtain a fused protein complex.

In one or more embodiments, the domain for protein-protein interaction comprises PDZ domain and the ligand thereof is PDZ ligand; the phenylalanine ammonia lyase and the 4-coumaryl-CoA ligase are separately fused to PDZ and PDZ ligand; preferably, the phenylalanine ammonia lyase is fused to PDZ and the 4-coumaryl-CoA ligase is fused to PDZ ligand.

In one or more embodiments, when the phenylalanine ammonia lyase is fused to PDZ, it also comprises an ER/K linker for connection (PAL-ER/K-PDZ).

In one or more embodiments, when the 4-coumaryl-CoA ligase is fused to PDZ ligand, it also comprises a linker (GGGGS)₂ for connection (PDZlig-(GGGGS)₂-4CL).

In one or more embodiments, the domain for protein-protein interaction comprises a domain selected from the group: PDZ domain, SH3 domain, WW domain, LIM domain, DD domain, PH domain, EH domain, GBD domain.

In one or more embodiments, the domain for protein-protein interaction comprises an SH3 domain, and its ligand is SH3 ligand; the phenylalanine ammonia lyase and 4-coumaryl-CoA ligase are respectively fused with the SH3 domain and its ligand; preferably, the phenylalanine ammonia lyase is fused with SH3 domain, the 4-coumaryl-CoA ligase is fused with SH3 ligand; more preferably, when the phenylalanine ammonia lyase is fused with SH3, it also comprises an ER/K linker for connection (PAL-ER /K-SH3); when the 4-coumaric acid coenzyme A is fused with SH3 ligand, it also comprises (GGGGS)₂ linker for connection (SH3lig-(GGGGS)z -4CL).

In one or more embodiments, when the phenylalanine ammonia lyase is fused with PDZ, the phenylalanine ammonia lyase is located at the N-terminus, and the PDZ is located at the C-terminus.

In one or more embodiments, when the 4-coumaryl-CoA ligase is fused with the PDZ ligand, the PDZ ligand is located at the N-terminus, and the 4-coumaryl-CoA ligase is located at the C-terminus.

In one or more embodiments, when the phenylalanine ammonia lyase is fused with SH3, the phenylalanine ammonia lyase is located at the N-terminus, and the SH3 is located at the C-terminus.

In one or more embodiments, when the 4-coumaryl-CoA ligase is fused with the SH3 ligand, the SH3 ligand is located at the N-terminus, and the 4-coumaryl-CoA ligase is located at the C-terminus.

In one or more embodiments, in (1), the prokaryotic cell also comprises exogenous genes encoding enzymes promoting malonyl-CoA production; preferably comprising matC, matB, ACS, FabF.

In one or more embodiments, when introduced into cells, the genes encoding PDZ ligand, 4-coumaryl-CoA ligase, phenylalanine ammonia lyase, ER/K, PDZ, flavone synthase I, chalcone synthase, and chalcone isomerase are located in a construct (plasmid).

In one or more embodiments, when introduced into cells, the genes encoding flavone 6-hydroxylase and cytochrome P450 oxidoreductase are located in a construct, preferably also comprising 2B1 (cytochrome P450 2B1 family soluble protein) gene.

In one or more embodiments, when introduced into cells, the genes encoding matC, matB, ACS, FabF are located in a construct.

In one or more embodiments, the "promoting" means statistically "promoting", such as promoting more than 5%, more than 10%, more than 20%, more than 50%, more than 80%, more than 100% or higher.

In one or more embodiments, when introduced into cells, the genes encoding SH3lig, 4-coumaryl-CoA ligase, phenylalanine ammonia lyase, ER/K, SH3 and chalcone synthase are located in a construct.

In one or more embodiments, when introduced into cells, the genes encoding chalcone isomerase, flavone synthase I are located in a construct.

In one or more embodiments, the prokaryotic cell is a cell with a substrate synthesis pathway of formula (I); preferably, the prokaryotic cell is an *Escherichia coli* cell.

In another aspect, the present disclosure provides a prokaryotic cell for synthesizing flavonoid compound, wherein it comprises exogenous genes encoding the following enzymes: phenylalanine ammonia lyase (PAL), 4-coumaryl-CoA ligase (4CL), chalcone synthase (CHS), chalcone isomerase (CHI) and flavone synthase I (FNSI); and, in the prokaryotic cell, the transmembrane protein rhodanese Ygap of *Escherichia coli* is up-regulated or a target gene or target gene combination selected from the following group is down-regulated: pyrB, accC, accB, purC, glyA, tktA, fabB, leuD, leuC, glpC, folk, leuA; the flavonoid compound is chrysin compound (chrysin or apigenin).

In one or more embodiments, the prokaryotic cell also comprises exogenous genes encoding the following enzymes: flavone 6-hydroxylase (F6H) and cytochrome P450 oxidoreductase (CPR); wherein the flavonoid compound is baicalein compound (such as baicalein or scutellarein).

In another aspect, the present disclosure provides a use of the prokaryotic cell for synthesizing a flavonoid compound; preferably, the flavonoid compound comprises: chrysin compound, baicalein compound.

In another aspect, the present disclosure provides a kit for producing a flavonoid compound, wherein the kit comprises the prokaryotic cell.

In one or more embodiments, the kit also comprises a cell culture medium.

In one or more embodiments, the kit also comprises a substrate of formula (I).

In one or more embodiments, in the kit.

In another aspect, the present disclosure provides a kit for constructing host cells for synthesizing flavonoid compounds, wherein the kit comprises: (a) a construct (for example, an expression construct) expressing phenylalanine ammonia lyase (PAL), 4-coumaryl-CoA ligase (4CL), chalcone synthase (CHS), chalcone isomerase (CHI) and flavone synthase I (FNSI); (b) a construct (for example, an expression construct) expressing transmembrane protein rhodanese Ygap of Escherichia coli; or a construct (for example, an expression construct) expressing a down-regulator, wherein the down-regulator down-regulates the target gene or target gene combination selected from the following group: pyrB, accC, accB, purC, glyA, tktA, fabB, leuD, leuC, glpC, folk, leuA; optionally, the kit further comprises a construct expressing genes encoding flavone 6-hydroxylase and cytochrome P450 oxidoreductase.

In another aspect, the present disclosure provides a use of regulators for promoting biosynthesis of flavonoid compounds, the regulators are selected from: (i) transmembrane protein rhodanese Ygap of *Escherichia coli* or an up-regulator thereof; or (ii) a down-regulator, wherein the down-regulator down-regulates the target gene or target gene combination selected from the following group: pyrB, accC, accB, purC, glyA, tktA, fabB, leuD, leuC, glpC, folk, leuA.

In one or more embodiments, the promoting biosynthesis of flavonoid compounds is promoting the synthesis of flavonoid compounds in prokaryotic cells.

Other aspects of the present disclosure will be apparent to those skilled in the art based on the disclosure herein.

### DESCRIPTION OF DRAWINGS

Figure 1. Comparison of chrysin production between strain J-1 and strain J-2 overexpressing ygaP.
Figure 2. Comparison of baicalein production between strain J-3 and strain J-4 overexpressing ygaP.
Figure 3. Comparison of baicalein production in engineered strain as a control and strains with various down-regulated target genes.

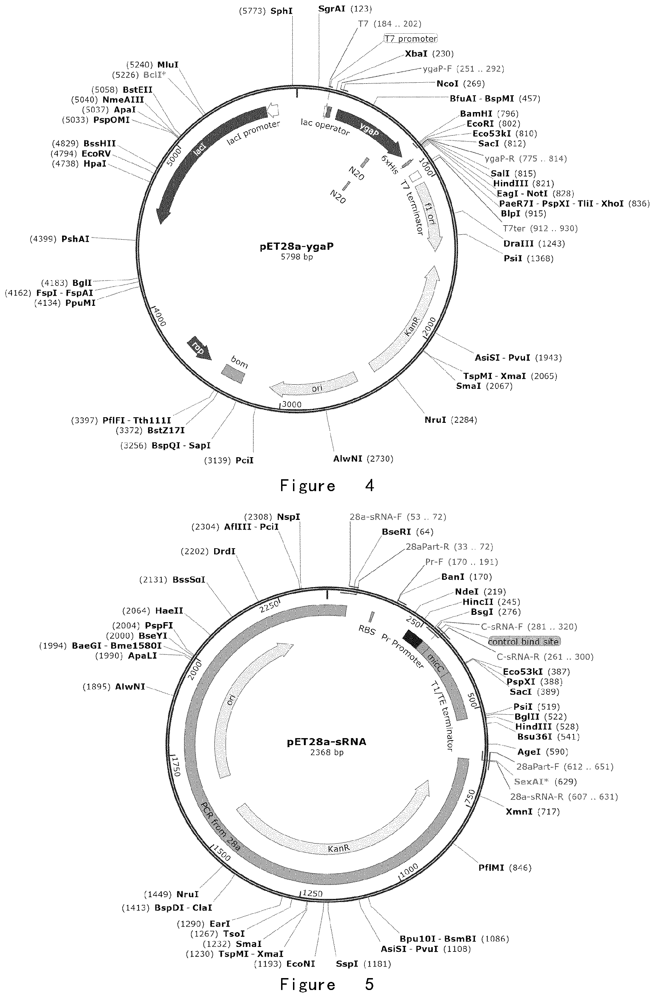

Figure 4. A schematic diagram of the plasmid structure of pET28a-ygaP.
Figure 5. A schematic diagram of the plasmid structure of PET28a-sRNA.

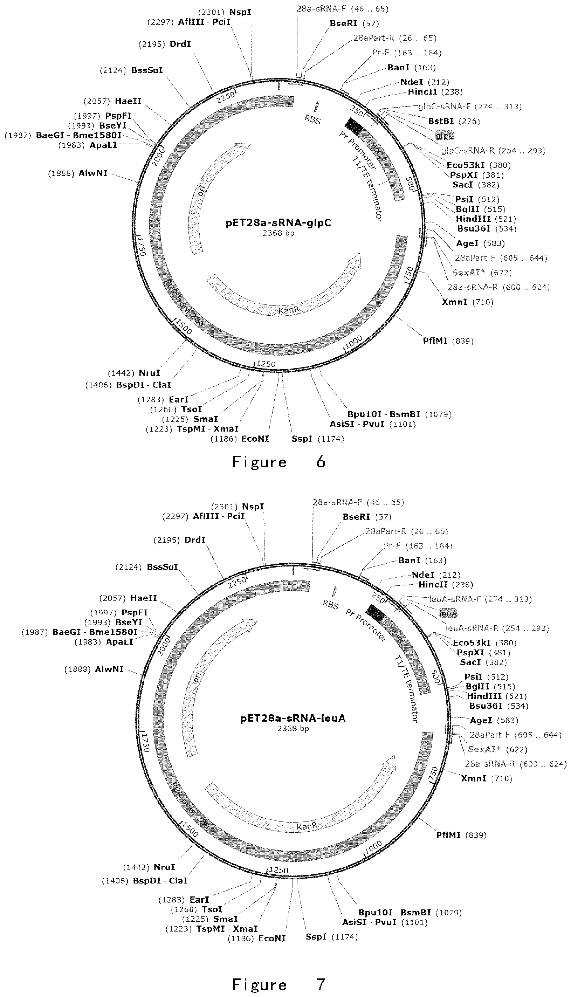

Figure 6. A schematic diagram of the plasmid structure of pET28a-sRNA-glpC.
Figure 7. A schematic diagram of the plasmid structure of pET28a-sRNA-leuA.

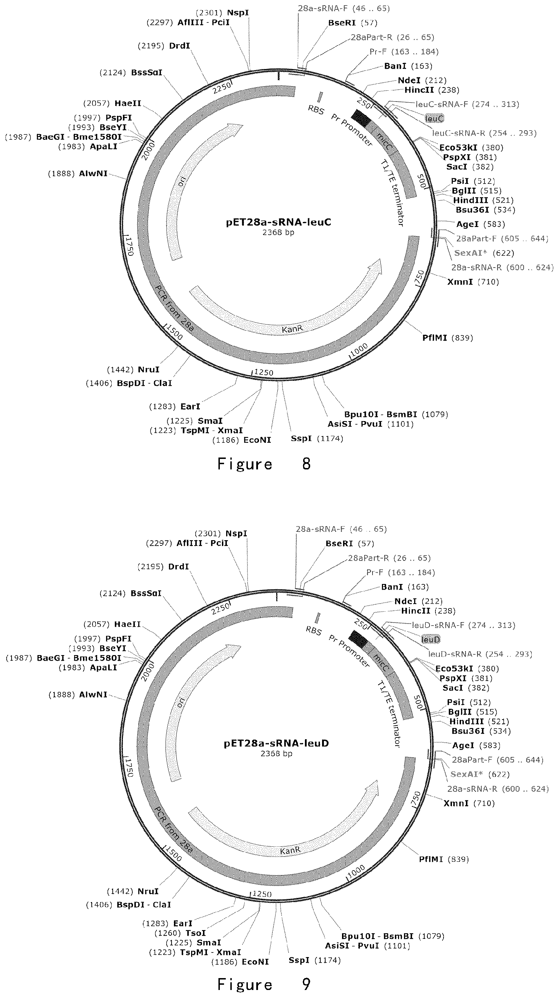

Figure 8. A schematic diagram of the plasmid structure of pET28a-sRNA-leuC.
Figure 9. A schematic diagram of the plasmid structure of pET28a-sRNA-leuD.

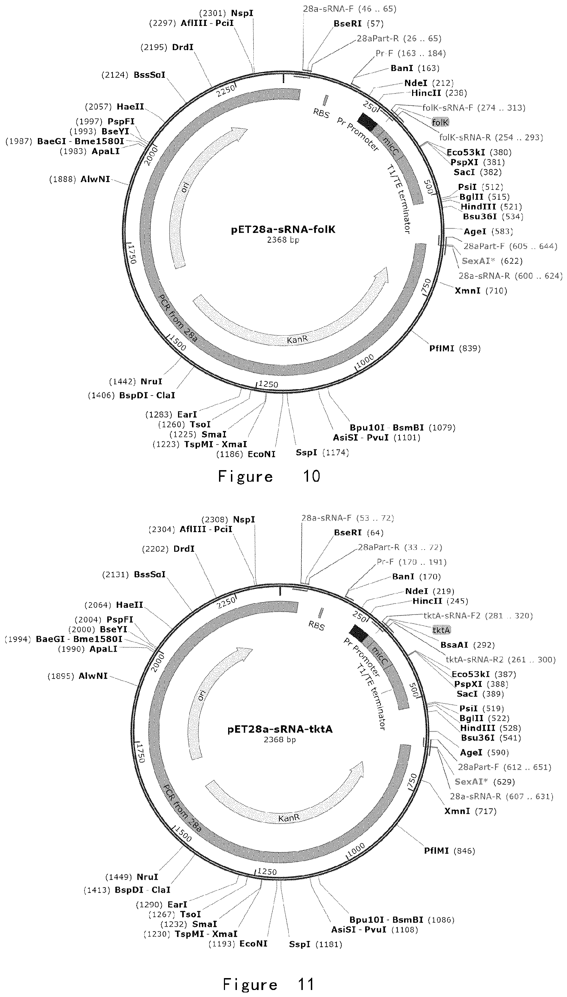

Figure 10. A schematic diagram of the plasmid structure of pET28a-sRNA-folK.
Figure 11. A schematic diagram of the plasmid structure of pET28a-sRNA-tktA.

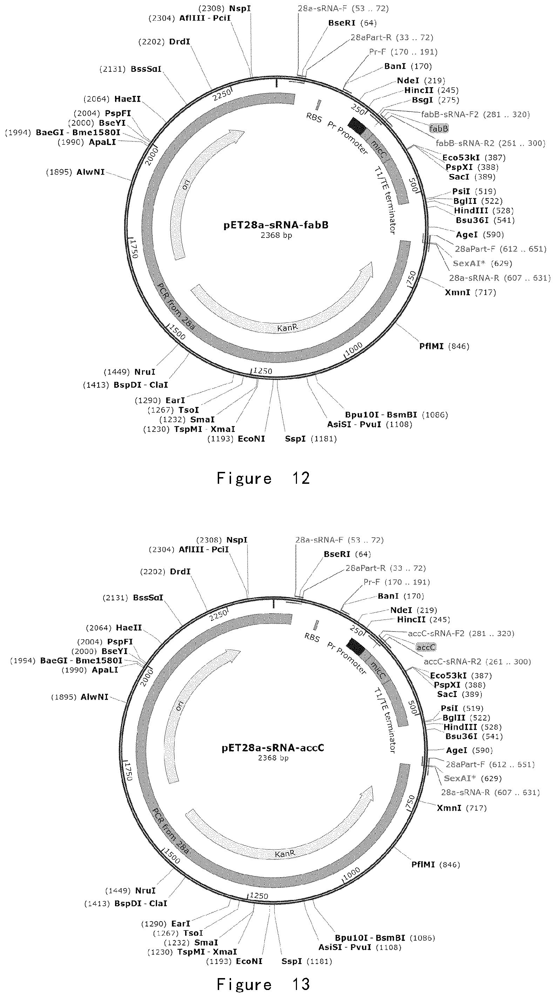

Figure 12. A schematic diagram of the plasmid structure of pET28a-sRNA-fabB.
Figure 13. A schematic diagram of the plasmid structure of pET28a-sRNA-accC.

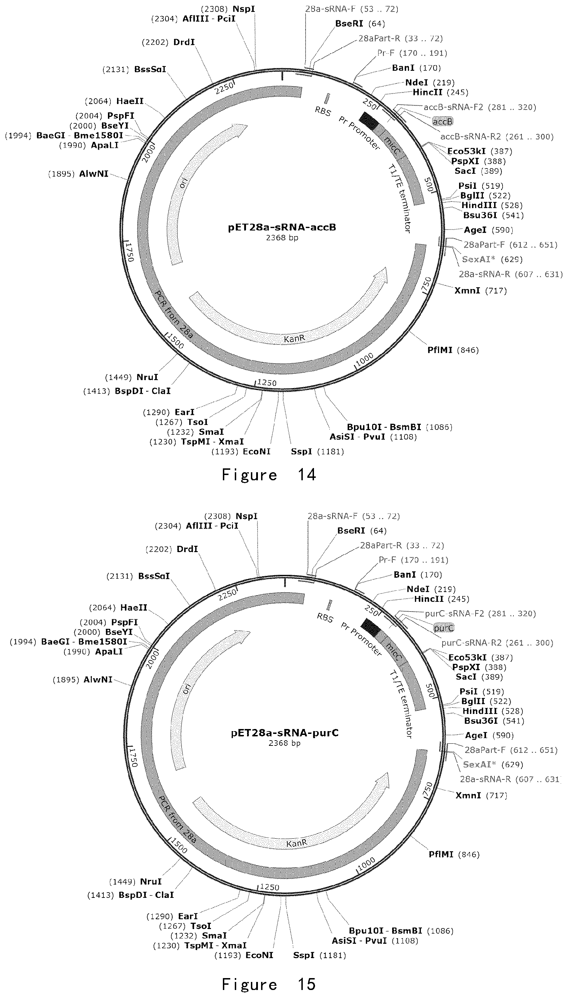

Figure 14. A schematic diagram of the plasmid structure of pET28a-sRNA-accB.
Figure 15. A schematic diagram of the plasmid structure of pET28a-sRNA-purC.

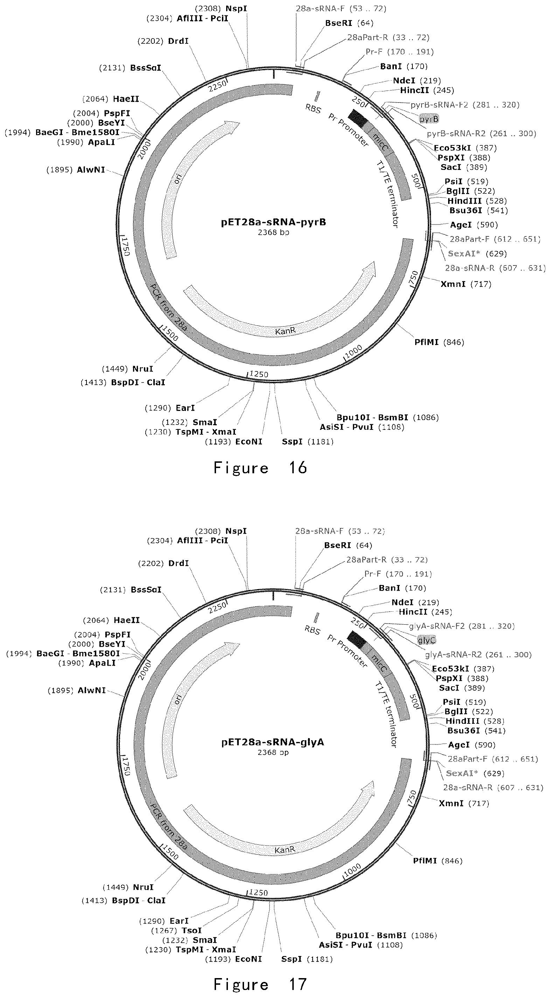

Figure 16. A schematic diagram of the plasmid structure of pET28a-sRNA-pyrB.
Figure 17. A schematic diagram of the plasmid structure of pET28a-sRNA-glyA.

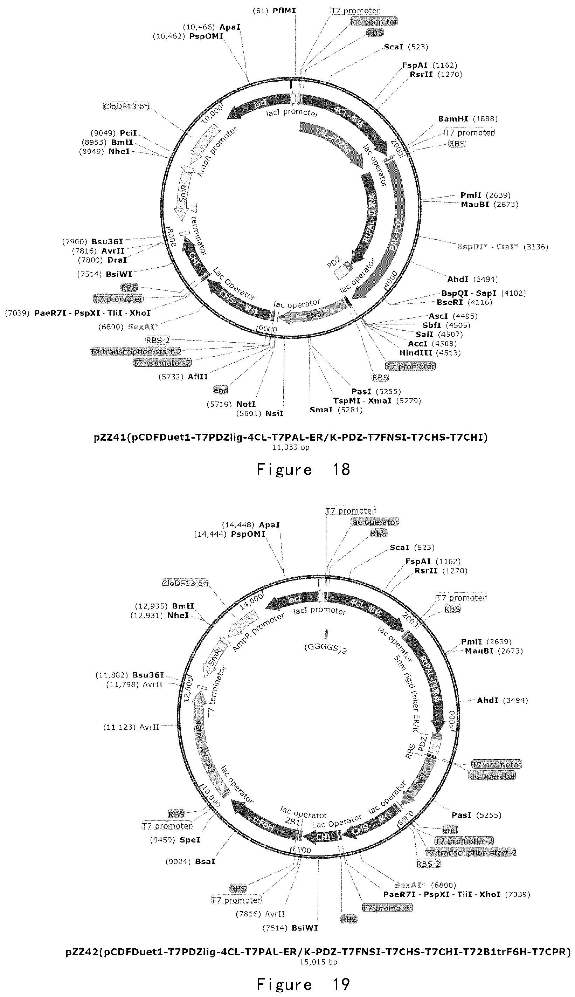

Figure 18. A schematic diagram of the plasmid structure of pZZ41.
Figure 19. A schematic diagram of the plasmid structure of pZZ42.
Figure 20. A schematic diagram of the plasmid structure of pYH38.
Figure 21. A schematic diagram of the synthesis pathway of baicalein and scutellarein production via fermentation using phenylalanine as a precursor.

### DETAILED DESCRIPTION

After in-depth researches, the present inventors provide a novel optimal strategy for the biosynthesis of flavonoid compounds (such as baicalein compounds/chrysin compounds). Through appropriate gene regulation in cells for production, a significant increase in the production of flavonoid compounds was achieved. The regulation comprises an up-regulation of transmembrane protein rhodanese Ygap of *Escherichia colian* or a down-regulation of one or more target genes (pyrB, accC, accB, purC, glyA, tktA, fabB, leuD, leuC, glpC, folK or leuA). The disclosure also discloses optimized host cells and a use thereof.

As used herein, the "flavonoid compound" is a compound with the following parent nucleus structure:

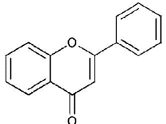

As used herein, the "chrysin (class) compound" comprises chrysin or variants, isomers, derivatives, precursors, salts, or others with its parent nucleus structure, for example, comprising chrysin or apigenin.

As used herein, the "baicalein (class) compound" comprises baicalein or variants, isomers, derivatives, precursors, salts, or others with its parent nucleus structure, for example, comprising baicalein or scutellarein.

As used herein, "exogenous" or "heterologous" refers to the relationship between two or more nucleic acid sequences or protein sequences from different sources, or the relationship between proteins/genes and host cells. For example, although the host cell itself may comprise a corresponding gene or produce a corresponding protein, synthetically/recombinantly established genes/proteins are "exogenous" or "heterologous" relative to the host cell when introduced into the host cell by genetic engineering methods.

As used herein, the "operably linked (linked)" or "operatively linked (linked)" refers to the functional spatial arrangement of two or more nucleic acid regions or nucleic acid sequences. For example: the promoter region is placed at a specific position relative to the nucleic acid sequence of the target gene, so that the transcription of the nucleic acid sequence is guided by the promoter region, thus, the promoter region is "operably linked" to the nucleic acid sequence.

As used herein, the term "construct (constructs)" or "expression construct (constructs)" refers to a recombinant DNA molecule comprising a desired nucleic acid coding sequence, wherein it may comprise one or more gene expression cassettes. The "construct (constructs)" is usually contained in an expression vector.

As used herein, the PAL, 4CL, CHS, CHI and FNSI proteins are proteins formed in the expression system by synthesis pathway of chrysin compounds or apigenin compounds.

As used herein, the F6H and CPR proteins are proteins that convert chrysin compounds or apigenin compounds into baicalein compounds or scutellarein compounds in an expression system.

As used herein, the matC, matB, ACS and/or FabF proteins are enzymes that promote malonyl-CoA production in an expression system.

The above-mentioned wild-type proteins or genes have been identified in the art, therefore, they can be obtained and prepared from public sources. As a preferable embodiment of the present disclosure, PAL is derived from *Rhodotorula toruloides* with the sequence shown in GenBank accession number AAA33883.1; 4CL is derived from *Petroselium crispum* with the sequence shown in GenBank accession number KF765780.1; CHS is derived from *Petunia X hybrida* with the sequence shown in GenBank accession number KF765781.1; CHI gene is derived from *Medicago sativa* with the sequence shown in GenBank accession number KF765782.1; FNS I is derived from *Petroselium crispum* with the sequence shown in Swiss-Prot accession number Q7XZQ8.1.

Wild-type F6H and CPR have also been identified in the prior art. As a preferable embodiment of the present disclosure, F6H is derived from *Scutellaria baicalensis* with the sequence shown in GenBank accession number ASW21050.1. As a preferable embodiment of the present disclosure, CPR is derived from *Arabidopsis thaliana* with the sequence shown in GenBank accession number NP_849472.2. As a preferable embodiment of the present disclosure, CPR is derived from *Arabidopsis thaliana,* with the sequence shown in GenBank accession number NP_849472.2.

Wild-type matC, matB, ACS, FabF proteins have also been identified in the prior art. As a preferable embodiment of the present disclosure, matC is derived from leguminous rhizobium (*Rhizobium leguminosarum*), with the sequence shown in GenBank accession number KF765784.1; matB is derived from leguminous rhizobium (*Rhizobium leguminosarum*), with the sequence shown in GenBank accession number AGZ04579.1; ACS is derived from *Escherichia coli* (*Escherichia coli*), with the sequence shown in GenBank accession number CP062211.1; FabF is derived from *Escherichia coli* (*Escherichia coli*), with the sequence shown in GenBank accession number AP023237.1.

Baicalein and scutellarein are two structurally similar and important flavonoid compounds. Baicalein has a molecular formula of C₁₅H₁₀O₅ with the molecular weight of 270.24. Scutellarein has a molecular formula of C₁₅H₁₀O₆ with the molecular weight of 286.24. Their structures are as follows:

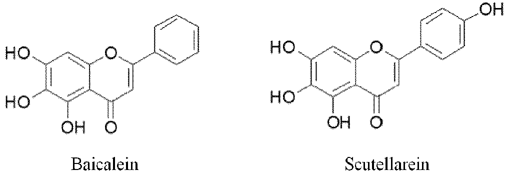

Chrysin is also a flavonoid compound. In enzyme-mediated biosynthetic pathways, chrysin is an upstream compound (intermediate compound) of baicalein or scutellarein, with the structural formula as shown below.

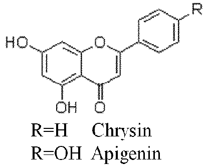

The inventors of the present disclosure are committed to studying a novel optimal strategy for biosynthesis of flavonoid compounds (such as baicalein compounds/chrysin compounds). After extensive researches, screening and experiments, the inventors have identified an optimized strategy that significantly enhances the yield of flavonoid compounds, including up-regulating the expression or activity of transmembrane protein rhodanese Ygap of *Escherichia coli* in host cells.

It should be understood that according to the functions of Ygap or signaling pathways thereof (preferably, it also comprises upstream genes and downstream genes), various methods well known to those skilled in the art can be used to regulate the expression or acitivity of Ygap or regulate the relevant upstream genes or downstream genes of Ygap. For example, various methods well known to those skilled in the art can be used to overexpress Ygap or upstream genes or downstream genes thereof.

In the present disclosure, the up-regulation of Ygap or an encoding gene thereof, an upstream or downstream protein or encoding genes thereof comprises using an up-regulator of Ygap protein or an encoding gene thereof. The upregulator comprises promoters, activators, agonists. The "up-regulation" and "promotion" comprises "up-regulation" and "promotion" of protein activity or "up-regulation" and "promotion" of protein expression, and they are "up-regulation" and "promotion" with statistical significance. Any substance that can increase the activity of Ygap or signaling pathway proteins comprising Ygap (including its upstream proteins and downstream proteins), increase the stability of Ygap or signaling pathway proteins comprising Ygap, upregulate the expression of Ygap or signaling pathway proteins comprising Ygap, increase the effective time of Ygap or signaling pathway proteins comprising Ygap and increase the phosphorylation/activation level of each protein can be used in the present disclosure as useful substances for up-regulating Ygap or signaling pathways thereof. They can be chemical compounds, small chemical molecules, biomolecules. The biomolecules can be nucleic acids (including DNA, RNA) or proteins.

The present disclosure also provides a method for up-regulating the expression of Ygap in cells, wherein the method comprises: transferring a gene encoding Ygap or an expression construct or vector comprising the encoding gene into the cells. Additionally, gain-of-function mutations can be introduced into Ygap or an encoding gene thereof. An expression-enhanced promoter or a tissue-specific promoter can be used to promote the expression of encoding gene of Ygap, or an enhancer can be used to promote the expression of encoding gene of Ygap. It should be understood that other methods for up-regulating the expression of Ygap in cells should also be included in the present disclosure.

The present disclosure also provides another optimal strategy for significantly increasing the yield of flavonoid compounds, including down-regulating target proteins or their encoding genes selected from the following group in host cells: pyrB (GenBank accession number CAD6022649.1), accC (GenBank accession number CAD6001830.1), accB (GenBank accession number CAD6001842.1), purC (GenBank accession number CAD6007197.1), glyA (GenBank accession number CAD6006651.1), tktA (GenBank accession number CAD6004304.1), fabB (GenBank accession number CAD6008062.1), leuD (GenBank accession number CAD6022253.1), leuC (GenBank accession number CAD6022253.1), glpC (GenBank accession number CAD6008699.1), folK (GenBank accession number CAD6022053.1), leuA (GenBank accession number CAD6022244.1). Agents that have a down-regulating effect on such target proteins or their encoding genes are called down-regulators.

In the present disclosure, the down-regulators of above target genes refer to any substance that can decrease the activity of target proteins, decrease the stability of target proteins or encoded genes thereof, down-regulate the expression of target proteins, decrease the effective time of target proteins, inhibit the transcription and translation of encoding genes of target proteins, and reduce phosphorylation/activation level of proteins. These substances can all be used in the present disclosure. They can be chemical compounds, small chemical molecules, biomolecules. The biomolecules can be nucleic acids (including DNA, RNA) or proteins. For example, the down-regulators are: interfering RNAs or antisense nucleotides that specifically interfering the expression of target genes, or a gene-editing agent specifically targeting the genes, and so on.

The present disclosure provides a method for down-regulating the expression of target genes in cells, comprising introducing an interfering molecule that interferes with the expression of target genes into the cells. As a more specific embodiment, the interfering molecule is sRNA. Another method for downregulating target gene expression in cells comprises targeted mutation, gene editing or gene recombination of the target genes. As a more specific embodiment, a CRISPR/Cas9 system can be used for gene editing, thereby knocking out or down-regulating the target genes. Appropriate sgRNA target sites will lead to higher gene editing efficiency. Therefore, before proceeding with gene editing, suitable target sites can be designed and found. After designing specific target sites, in vitro screening for cell activity is also required to obtain effective target sites for subsequent experiments. It should be understood that methods for down-regulating target genes/target proteins in cells are not limited to those listed above.

As a preferable embodiment of the present disclosure, the inventors use enzyme assembly technology to produce baicalein or scutellarein by fermentation. The principle of this method is to use interacting protein pairs (such as PDZ and PDZ ligand) to fuse with the enzymes PAL and 4CL in synthesis pathways of baicalein, allowing PAL and 4CL to self-assemble within *E. coli,* forming a dual-enzyme complex reactor, thereby enhancing the yield of the target compound. The inventors first discovered that constructing PAL and 4CL into a complex (complex reactor) in a prokaryotic expression system for synthesizing baicalein compounds/chrysin compounds can significantly increase the system's yield. Any biological material or technique that enables the formation of an active complex of PAL and 4CL can be applied in this disclosure. As a preferable embodiment of the present disclosure, the domain for protein-protein interaction comprises a domain selected from the group: PDZ domain, SH3 domain, WW domain, LIM domain, DD domain, PH domain, EH domain. As a more preferable embodiment of the present disclosure, the domain for protein-protein interaction can comprise a domain selected from the following group: PDZ domain, SH3 domain; and their corresponding ligands are PDZ ligand (PDZlig) or SH3 ligand (SH3lig).

Protein-protein interactions are primarily mediated efficiently by protein domains. PDZ, SH3, WW and other domains can recognize and bind a conserved short peptide sequence of ligand through one or more protein binding "pockets". For the PDZ domain, it usually binds the 4-5 amino acid residues of C-terminal of the ligand. It can also bind to the intermediate sequences of the ligand, polymerizing with itself or other domains or binding to lipids on the membrane.

In the present disclosure, other methods that construct a complex of PAL and 4CL and retain the biological activity of PAL and 4CL can also be included, such as fusing them to form a fused protein with a suitable spatial structure. The activity of fused protein can be determined by experimental testing. The fusion between PAL and 4CL can be a direct connection or a linker (Linker) can be used for connection.

As another optional embodiment of the present disclosure, aroG, especially aroG^{fbr} (an aroG gene with a mutation from G to A at position 436), and pheA, especially pheA^{ftr} (a pheA gene (pheA^{fbr}) with a mutation from A to C at position 976) can be over-expressed in the prokaryotic expression system to construct a high-yield prokaryotic expression system for phenylalanine. By introducing exogenous synthesis pathway of baicalein or scutellarein compounds/chrysin compounds into this system, the strain can utilize glucose to synthesize baicalein/chrysin compounds de novo.

In the present disclosure, the prokaryotic cell also comprises exogenous genes encoding enzymes promoting malonyl-CoA production; preferably comprising matC, matB, ACS, FabF.

In the present disclosure, the prokaryotic expression system (prokaryotic cell) is a cell with synthetic pathway for the substrate of formula (I). Commonly used prokaryotic expression systems comprise *Escherichia coli, Bacillus subtilis,* etc.; for example, *Escherichia coli* cells (E. *coli*), such as *Escherichia coli* BL21 (DE3).

In addition to the preferred proteins listed in this present disclosure (including the wild-type and mutant proteins), the disclosure also comprises their analogs. The difference between these analogs and the original protein may be differences in amino acid sequences, differences in modifications that do not affect the sequence, or both. These proteins include natural or induced genetic variants. Induced variants can be obtained by various techniques, such as random mutagenesis by radiation or exposure to mutagens, site-directed mutagenesis or other techniques known in molecular biology. Analogues also include analogs with residues that differ from natural L-amino acids (e.g., D-amino acids), as well as analogs with non-naturally occurring or synthetic amino acids (e.g., β, γ-amino acids). It should be understood that the proteins of the present description are not limited to the representative proteins exemplified above.

Based on the preferred proteins listed in the present disclosure (including the wild-type and mutant proteins), the disclosure also comprises proteins having high homology (e.g., having 70% or higher homology to the specific protein sequences listed; preferably 80% or higher; more preferably 90% or higher, such as 95%, 98%, or 99% homology) to the listed proteins, and having similar or identical functions to the protein are also included within the scope of this disclosure.

Proteins or genes from specific species are listed in the present disclosure. It should be understood that although the present disclosure preferably studies proteins or genes obtained from a specific species, but other proteins or genes obtained from other species that are highly homologous (such as more than 60%, such as 70%, 80%, 85%, 90%, 95%, or even 98% identical in sequence) to the protein or gene are also within the scope of the present disclosure.

The present disclosure also provides a polynucleotide sequence encoding the protein or a conservative variant protein thereof in the present disclosure. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strand DNAs. The polynucleotide encoding the mature protein of variant in the present disclosure comprises: the coding sequence encoding only the mature protein; the coding sequence of the mature protein and various additional coding sequences; the coding sequence of the mature protein (and optional additional coding sequences) and non-coding sequence.

The present disclosure also comprises polynucleotide sequences formed by codon optimization of the aforementioned gene sequences, for example, by codon optimization according to the preferences of the host cells.

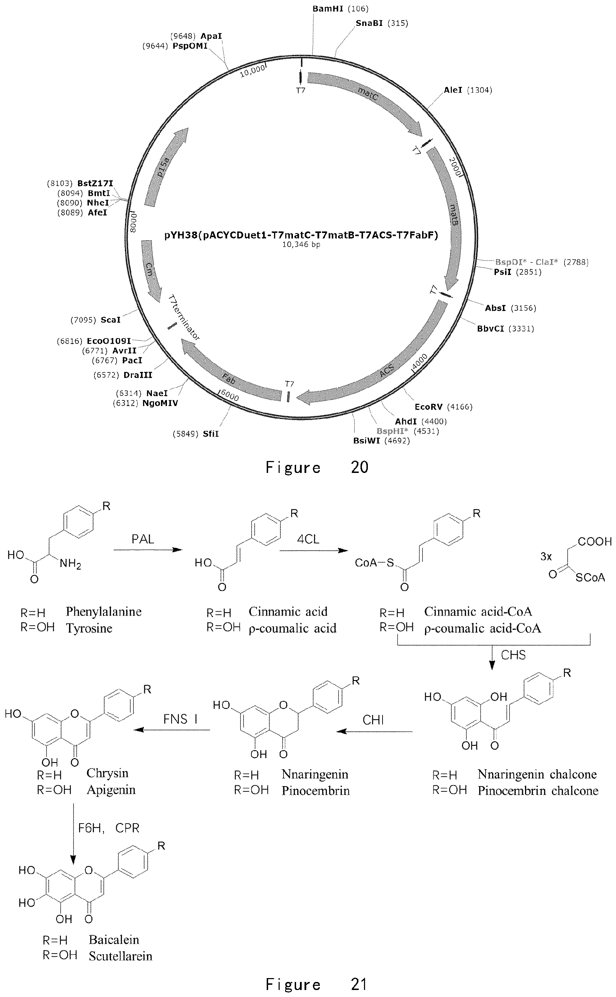

In the present disclosure, an engineering strain with high yield of baicalein or scutellarein compounds is constructed, comprising exogenous encoding genes of the following enzymes: F6H, CPR, PAL, 4CL, CHS, CHI and FNSI; and, the engineering strain comprises an encoding gene of exogenous transmembrane protein rhodanase Ygap of *Escherichia coli;* or a target gene or target gene combination selected from the following groups is down-regulated in the engineering strain: pyrB, accC, accB, purC, glyA, tktA, fabB, leuD, leuC, glpC, folK, leuA. Preferably, after the enzyme is expressed, a complex (complex reactor) is constructed by PAL and 4CL. By cultivating the engineered strains and using phenylalanine or tyrosine as substrates, baicalein or scutellarein compounds can be produced. Production with phenylalanine or tyrosine as a substrate is suitable for large-scale production of compounds. A schematic diagram of the synthesis pathway of baicalein and scutellarein production via fermentation using phenylalanine as a precursor is shown in Figure 21.

In the present disclosure, an engineering strain with high yield of chrysin compounds is constructed, comprising exogenous encoding genes of the following enzymes: PAL, 4CL, CHS, CHI and FNSI; and, the engineering strain comprises an encoding gene of exogenous transmembrane protein rhodanase Ygap of *Escherichia coli;* or a target gene or target gene combination selected from the following groups is down-regulated in the engineering strain: pyrB, accC, accB, purC, glyA, tktA, fabB, leuD, leuC, glpC, folK, leuA. Preferably, after the enzyme is expressed, a complex (complex reactor) is constructed by PAL and 4CL. By cultivating the recombinant strain and using phenylalanine or tyrosine as substrates, baicalein compounds or scutellarein compounds can be produced. Production with phenylalanine or tyrosine as a substrate is suitable for large-scale production of compounds.

As a preferable embodiment of the present disclosure, the F6H further comprises a polypeptide tag fused thereto, for example, the polypeptide tag can be selected from: 8RP, Sumo, MBP, 2B1, or a combination thereof; preferably 2B1. A peptide linker may or may not be included between the polypeptide tag and the F6H, and the peptide linker does not affect the biological activity of the two. F6H is linked to 2B 1, and an improved F6H mutant 2B1trF6H can be obtained.

It is also possible to further introduce upstream pathways of the above-mentioned substrates (phenylalanine or tyrosine) into the above-mentioned engineering strain, for example, comprising: producing phenylalanine or tyrosine from glucose or glycerol through glycolysis, pentose phosphate pathway or shikimic acid pathway. It is to be understood that methods based on such pathways to form phenylalanine or tyrosine are also included by the present disclosure. Methods of enhancing the pathways to form phenylalanine or tyrosine by methods known in the art are included by the present disclosure.

As a preferable embodiment, exogenous aroG, especially its aroG^{fbr}, and pheA, especially its pheA^{fbr}, can be further introduced into the above-mentioned engineering strain using phenylalanine or tyrosine as a substrate to obtain another recombinant strain, wherein the recombinant strain can use glucose as a substrate to produce baicalein compounds/chrysin compounds. The production with glucose as the substrate has low cost and is very suitable for large-scale production of compounds.

On the basis of establishing the optimized expression system of the present disclosure and using it for production, those skilled in the art can also systematically study a series of factors to improve the yield of baicalein, scutellarein compounds or chrysin compounds, including gene efficiency and suitability, gene dosage and culture medium. In addition, the yield of the target compound can also be increased by scaling up the production. For example, based on the yield obtained under shake flask and simple culture conditions, the yield can typically increase by 2 to 1000 times or more when further scaling up production, implementing fed-batch culture (this culture can continuously provide abundant substrates), or providing optimal fermenter-level production conditions (such as the control of optimized temperature, optimized dissolved oxygen, etc.). These operations and optimization methods should also be included in the present disclosure. It can be expected that, in the recombinant prokaryotic cells of the present disclosure, in some optimized equipment and operating processes, the amount of the target product will further increase.

After products are obtained from fermentation, methods known in the art can be used to extract the target compound from the product. The product can be analyzed and identified using known methods such as high-performance liquid chromatography to confirm that the desired compound is obtained.

The strain of the disclosure has good stability and can realize large-scale cultivation and production of baicalein or scutellarein compounds/chrysin compounds in a bioreactor. The yield of the target compound of the preferable strain of the present disclosure is very high.

Compared with traditional plant extraction methods, microbial fermentation has several advantages, such as more fast extraction, less affection by external factors and so on. Also, the yield of some compounds synthesized by microorganisms is much higher than that of plant extraction. Microbial fermentation has become an important method of obtaining natural products. In the present disclosure, *Escherichia coli* used for production of baicalein or scutellarein compounds/chrysin compounds is able to achieve more economical and convenient manufacture of the target compound.

The present disclosure also provides a kit comprising the engineering strain for producing flavonoid compounds. In addition, the kit also includes the culture medium of prokaryotic cells, substrates for synthesis such as phenylalanine, tyrosine or glucose, reagents for separating or detecting baicalein or scutellarein compounds. More preferably, the kit further comprises instructions for explaining methods for biosynthesizing flavonoid compounds.

The present disclosure also provides a kit for constructing the engineering strain for producing flavonoid compounds. The kit may include a series of constructs, for example, constructs provided in the examples of the present disclosure, or other constructs comprising the genes but with different gene arrangements or tandems. Expression vectors (expression constructs) can be constructed using methods familiar to those skilled in the art. After knowing the enzyme to be selected and the cell system to be expressed, those skilled in the art can establish the expression construct. Gene sequences can be inserted into different expression constructs (such as expression vectors) or into the same expression construct, as long as the encoded polypeptide can be effectively expressed and active after being transferred into cells. In addition, the kit also includes prokaryotic cells, culture medium of prokaryotic cells, substrates for synthesis such as phenylalanine, tyrosine or glucose and reagents for separating or detecting baicalein or scutellarein compounds. More preferably, the kit further comprises instructions for explaining methods for biosynthesizing flavonoid compounds.

The disclosure is further described below in conjunction with specific embodiments. It should be understood that these examples are merely for illustrative purposes and not intended to limit the scope of the disclosure. The experimental methods without specifying the specific conditions in the following examples generally used the conventional conditions, such as those described in J. Sambrook, Molecular Cloning: A Laboratory Manual (3rd ed. Science Press) or followed the manufacturer's recommendation.

### 1. Experimental Materials

Polymerase chain reaction (PCR) gel recovery kit and plasmid extraction kit are from Axygen in the United States; polymerase chain reaction (PCR) high-fidelity enzyme PrimeSTAR Max DNA Polymerase is from TAKARA; restriction enzymes are all from NEB.

Standard compounds, baicalein and scutellarein, were purchased from Shanghai Yuanye Biotechnology Co., Ltd. Other reagents are analytical grade reagent or chromatographic grade reagent, purchased from Sinopharm Chemical Reagent Co., Ltd.

### 2. Bacterial strains and plasmids in the present disclosure

*Escherichia coli* DH10B was used for gene cloning, and *Escherichia coli* BL21 (DE3) strain was used for protein expression and production of baicalein and scutellarein.
pCDFDuet-1, pET28a, pACYCDuet-1 vectors were used for metabolic gene assembly. Sequence of 5nm rigid linker ER/K:
KAKLKEEEERKQREEEERIKRLEELAKRKEEERKGT (SEQ ID NO: 14).

### 3. Protein/gene/sRNA/structural domains

Wild-type proteins or genes specifically exemplified in the examples have been identified in the art. Therefore, they can be obtained and prepared from public sources, as shown in Table 1.

**Table 1**

| **Protein/ gene** | **Source species** | **Sequences or Accession number** |
|---|---|---|
| PAL | *Rhodotorula toruloides* | GenBank accession number AAA33883.1(RtPAL) |
| 4CL | *Petroselium crispum* | GenBank accession number KF765780.1(Pc4CL) |
| CHS | *Petunia X hybrida* | GenBank accession number KF765781.1 |
| CHI | *Medicago sativa* | GenBank accession number KF765782.1 |
| FNS I | *Petroselium crispum* | Swiss-Prot accession number Q7XZQ8.1 |
| F6H | *Scutellaria baicalensis* | GenBank accession number ASW21050.1 |
| CPR | *Arabidopsis thaliana* | GenBank accession number NP 849472.2 |
| | | |
| PDZ domain | Mouse α-syntrophin (syn) | Amino acids at position 77-171 in the sequence accession with GenBank number EDL06069 |
| matB | *Rhizobium leguminosarum* | GenBank accession number AGZ04579.1 |
| matC | *Rhizobium leguminosarum* | GenBank accession number KF765784.1 |
| ACS | *Escherichia coli* | GenBank accession number CP062211.1 |
| FabF | *Escherichia coli* | GenBank accession number AP023237.1 |
| PDZ ligand | GVKESLV(SEQ ID NO: 15) | |
| SH3 domain | AEYVRALFDFNGNDEEDLPFKKGDILRIRDKPEEQWWNAEDSEGKRGMIPVPYVEKY( SEQ ID NO: 16) | |
| SH3lig | PPPALPPKRRR(SEQ ID NO: 17) | |
| vgaP gene | *Escherichia coli* | GenBank accession number CAD6006090.1 |
| sRNA | tttctgttgggccattgcattgccactgattttccaacatataaaaagacaagcccgaacagtcgtccgggctttttttctcgag (SEO ID NO: 1) | |
| sRNA-glpC | gttttcgaagctggtgtcgttcattttctgttgggccattgcattgccactgattttccaacatataaaaagacaagcccgaac agtcgtccgggctttttttctcgag (SEQ ID NO: 2) | |
| sRNA-leuA | gaaaataatgacttgctggctcattttctgttgggccattgcattgccactgattttccaacatataaaaagacaagcccgaac agtcgtccgggctttttttctcgag (SEQ ID NO: 3) | |
| sRNA-leuC | tttttcgtataacgtcttagccattttctgttgggccattgcattgccactgattttccaacatataaaaagacaagcccgaacagt cgtccgggctttttttctcgag (SEQ ID NO: 4) | |
| sRNA-leuD | gtgtttgataaatttctctgccattttctgttgggccattgcattgccactgattttccaacatataaaaagacaagcccgaacagtc gtccgggctttttttctcgag (SEQ ID NO: 5) | |
| sRNA-folK | tatggcaatatacgccactgtcattttctgttgggccattgcattgccactgattttccaacatataaaaagacaagcccgaacag tcgtccgggctttttttctcgag (SEQ ID NO: 6) | |
| sRNA-tktA | ggcaagctctttacgtgaggacattttctgttgggccattgcattgccactgattttccaacatataaaaagacaagcccgaacag tcgtccgggctttttttctcgag (SEQ ID NO: 7) | |
| sRNA-fabB | gccagtaatcactgcacgtttcattttctgttgggccattgcattgccactgattttccaacatataaaaagacaagcccgaacagt cgtccgggctttttttctcgag (SEQ ID NO: 8) | |
| sRNA-accC | ggcaataacaattttatccagcattttctgttgggccattgcattgccactgattttccaacatataaaaagacaagcccgaacag tcgtccgggctttttttctcgag (SEQ ID NO: 9) | |
| sRNA-accB | tttttaatcttacgaatatccattttctgttgggccattgcattgccactgattttccaacatataaaaagacaagcccgaacagtcg ccgggctttttttctcgag (SEQ ID NO: 10) | |
| sRNA-purC | atacaactcagcttgcttttgcattttctgttgggccattgcattgccactgattttccaacatataaaaagacaagcccgaacagt cgtccgggctttttttctcgag (SEQ ID NO: 11) | |
| sRNA-pyrB | tttctgatatagcggattagccattttctgttgggccattgcattgccactgattttccaacatataaaaagacaagcccgaacagt cgtccgggctttttttctcgag (SEQ ID NO: 12) | |
| pyrB sRNA-glyA | aatgttcatttcacgctttaacattttctgttgggccattgcattgccactgattttccaacatataaaaagacaagcccgaacagtc gtccgggctttttttctcgag (SEQ ID NO: 13) | |

### 4. Plasmid construction

A plasmid containing a single gene was constructed, as shown in Table 2.

**Table 2**

| | **Recombinant plasmid** | **Construction** |
|---|---|---|
| 1 | pET28a-ygaP (Figure 4) | The ygaP gene was cloned from the genome of BL21(DE3), with an NcoI restriction site added to the 5' end and a BamHI restriction site added to the 3' end. The chosen vector backbone was pET28a, into which the ygaP gene was ligated at the NcoI and BamHI sites to obtain pET28a-T7 y gaP. |
| 2 | PET28a-sRNA (Figure 5) | The sRNA sequence was designed into the primers, and PCR was performed using pET28a as the template. The PCR product was cloned in one step and transformed into *E. coli* DH10B to obtain pET28a-sRNA. |
| 3 | pET28a-sRNA-glpC (Figure 6) | The sRNA-glpC sequence was designed into the primers, and PCR was performed using PET-28a-sRNA as the template. The PCR product was transformed into *E. coli* DH10B to obtain pET28a-sRNA via self-assembly of the plasmid. |
| 4 | pET28a-sRNA-leuA (Figure 7) | The sRNA-leuA sequence was designed into the primers, and PCR was performed using PET-28a-sRNA as the template. The PCR product was transformed into *E. coli* DH10B to obtain pET28a-sRNA-leuA via self-assembly of the plasmid. |
| 5 | pET28a-sRNA-leuC (Figure 8) | The sRNA-leuC sequence was designed into the primers, and PCR was performed using PET-28a-sRNA as the template. The PCR product was transformed into *E. coli* DH10B to obtain pET28a-sRNA-leuC via self-assembly of the plasmid. |
| 6 | pET28a-sRNA-leuD (Figure 9) | The sRNA-leuD sequence was designed into the primers, and PCR was performed using PET-28a-sRNA as the template. The PCR product was transformed into *E. coli* DH10B to obtain pET28a-sRNA-leuD via self-assembly of the plasmid. |
| 7 | pET28a-sRNA-folK (Figure 10) | The sRNA-folK sequence was designed into the primers, and PCR was performed using PET-28a-sRNA as the template. The PCR product was transformed into *E. coli* DH10B to obtain pET28a-sRNA-folK via self-assembly of the plasmid. |
| 8 | pET28a-sRNA-tktA (Figure 11) | The sRNA-tktA sequence was designed into the primers, and PCR was performed using PET-28a-sRNA as the template. The PCR product was transformed into *E. coli* DH10B to obtain pET28a-sRNA-tktA via self-assembly of the plasmid. |
| 9 | pET28a-sRNA-fabB (Figure 12) | The sRNA-fabB sequence was designed into the primers, and PCR was performed using PET-28a-sRNA as the template. The PCR product was transformed into *E. coli* DH10B to obtain pET28a-sRNA-fabB via self-assembly of the plasmid. |
| 10 | pET28a-sRNA-accC (Figure 13) | The sRNA-accC sequence was designed into the primers, and PCR was performed using PET-28a-sRNA as the template. The PCR product was transformed into *E. coli* DH10B to obtain pET28a-sRNA-accC via self-assembly of the plasmid. |
| 11 | pET28a-sRNA-accB (Figure 14) | The sRNA-accB sequence was designed into the primers, and PCR was performed using PET-28a-sRNA as the template. The PCR product was transformed into *E. coli* DH10B to obtain pET28a-sRNA-accB via self-assembly of the plasmid. |
| 12 | pET28a-sRNA-purC (Figure 15) | The sRNA-purC sequence was designed into the primers, and PCR was performed using PET-28a-sRNA as the template. The PCR product was transformed into *E. coli* DH10B to obtain pET28a-sRNA-purC via self-assembly of the plasmid. |
| 13 | pET28a-sRNA-pyrB (Figure 16) | The sRNA-pyrB sequence was designed into the primers, and PCR was performed using PET-28a-sRNA as the template. The PCR product was transformed into *E. coli* DH10B to obtain pET28a-sRNA-pyrB via self-assembly of the plasmid. |
| 14 | pET28a-sRNA-glyA (Figure 17) | The sRNA-glyA sequence was designed into the primers, and PCR was performed using PET-28a-sRNA as the template. The PCR product was transformed into *E. coli* DH10B to obtain pET28a-sRNA-glyA via self-assembly of the plasmid. |

A plasmid containing multiple genes was constructed, as shown in Table 3.

**Table 3**

| | **Recombinant plasmid** | **Construction** |
|---|---|---|
| 1 | pZZ41(pCDFDuet1-T7PDZlig-4CL-T7PAL-ER/K-PDZ-T7FNSI -T7CHS-T7CHI) (Figure 18) | Using Over-Lap PCR method, PAL, 5nm rigid linker ER/K and PDZ were fused to obtain PAL-ER/K-PDZ, with a BamHI restriction site added to the 5' end and an EcoRI restriction site added to the 3' end of PAL-ER/K-PDZ, then it was inserted into pCDFDuet-1 to obtain pCDFDuet1-T7PAL-ER/K-PDZ. |
| | | The PDZ ligand and (GGGGS)₂ sequence were designed into the upstream primer, with an NcoI restriction site added to the 5' end, and a BamHI restriction site to the 3' end of the 4CL sequence. PDZlig-4CL fused gene was obtained by PCR and then constructed into the NcoI and BamHI sites of pCDFDuet-1, resulting in pCDFDuet1-T7PDZlig-4CL. Using pCDFDuet1-T7PAL-ER/K-PDZ as a template, PCR was performed, and the PAL-ER/K-PDZ gene was constructed into the BamHI and EcoRI sites of the pCDFDuet1-T7PDZlig-4CL plasmid, yielding pCDFDuet 1- T7PD Zlig -4CL- T7P AL- ERIK - PDZ (PDZlig-4CL with a (GGGGS)₂ sequence between the two elements). |
| | | The T7FNSI-T7CHS-T7CHI gene was excised from the pYH57 plasmid by HindIII and AvrII double digestion (Production of plant-specific flavones baicalein and scutellarein in engineered E. coli from available phenylalanine and tyrosine), then ligated into the HindIII and AvrII sites of the previously mentioned plasmid, resulting in pZZ41 (pCDFDuetl-T7PDZlig-4CL-T7PAL-ER/K-PDZ-T7FNSI-T7CHS-T7CHI). |
| 2 | pZZ42(pCDFDuet1-T7PDZlig -4CL-T7PAL-ER/K-PDZ-T7FNSI -T7CHS-T7CHIT72B1trF6H-T7CPR) (Figure 19) | The T7 2B1trF6H-T7CPR gene was excised from pETDuet1-T7 2B1trF6H-T7CPR by digestion (Production of plant-specific flavones baicalein and scutellarein in an engineered E. coli from available phenylalanine and tyrosine). |
| | | Using PCR, the T7 2B1trF6H-T7CPR gene was amplified and then inserted into the AvrII site of pZZ41, resulting in pZZ42 (pCDFDuetl-T7PDZlig-4CL-T7PAL-ER/K-PDZ-T7FNSI-T7CHS-T7CHI-T7 2B1trF6H-T7CPR). |
| 3 | pYH38(pACYCDuet1-T7matC -T7matB-T7ACS-T7FabF (Figure 20) | Using PCR, the matC gene and matB gene were amplified. An NcoI restriction site was added to the 5' end and a HindIII restriction site to the 3' end of the matC gene. The matC gene was then ligated into the NcoI and HindIII sites of pACYCDuet-1 (comprising a T7 promoter before this site), resulting in pACYCDuet1-T7matC. |
| | | Similarly, the T7matB gene was obtained by PCR, with a HindIII site added to the 5' end and an Acc65I site to the 3' end. |
| | | Similarly, the T7ACS gene was obtained by PCR, with an Acc65I site added to the 5' end and a NotI site to the 3' end. |
| | | Similarly, the T7FabF gene was obtained by PCR, with a NotI site added to the 5' end and a XbaI site to the 3' end. |
| | | The T7matB gene was ligated into the HindIII and Acc65I sites of pACYCDuet1-T7matC. The T7ACS gene was then inserted into the Acc65I and NotI sites, followed by the insertion of the T7FabF gene into the NotI and XbaI sites. This resulted in the plasmid pACYCDuet1-T7matC-T7matB-T7ACS-T7FabF. |

### 5. Escherichia coli shake flask fermentation of chrysin and baicalein

Preparation for strain fermentation: The constructed plasmids were transformed into *E. coli* BL21(DE3). After inverted incubation at 37°C for 12 hours, positive clones were selected and inoculated into 2 mL of LB medium comprising appropriate antibiotic, then incubated at 37°C and 250 rpm for 10 hours to prepare seed culture for fermentation.

1% of the seed culture was transferred to 10 mL of MOPS medium comprising 2% glucose (with appropriate antibiotic added in the medium), cultivated at 37°C and 250 rpm until the strain OD=0.5, then 0.5 mM IPTG and 500 mg/L phenylalanine added, fermented at 22°C and 250 rpm for 3 days. 1 mL of the bacterial culture was taken and disrupted ultrasonically for 3 times, then mixed with an equal volume of ethyl acetate for twice extraction. After 12000rpm, 2min centrifugation, the organic phase was transferred to a new tube, then evaporated at room temperature or 30°C using a rotary evaporator. The residue was re-dissolved in 200 µL of methanol (5-fold concentration), mixed thoroughly and centrifuged at 12000rpm for 2min, with the supernatant transferred for HPLC detection.

### 6. HPLC detection

Conditions for liquid phase detection: solvent A: 0.1% formic acid in water, solvent B: acetonitrile; Gradient separation: 20%-55% solvent B for 0-20min, 55%-100% solvent B for 20-22min, 100% solvent B for 22-27min, 100%-20% solvent B for 27-35min and 20% solvent B for 35-40min; detected wavelength: 340nm, column temperature: 30°C.

Chromatographic column: Thermo syncronis C18 reversed-phase column (250mm×4.6mm, 5µm).

### Example 1. Chrysin production by fermentation of engineered strains

### 1. Strain (J-1)

The plasmids pZZ41 (pCDFDuet1-T7PDZlig-4CL-T7PAL-ER/K-PDZ-T7FNSI-T7CHS-T7CHI), pYH38 (pACYCDuet1-T7matC-T7matB-T7ACS-T7FabF) and pET28a were transformed into BL21(DE3) to obtain the engineered strain J-1, used for chrysin fermentation with phenylalanine as a precursor.

### 2. Strain with ygaP overexpression (J-2)

The plasmids pZZ41(pCDFDuet1-T7PDZlig-4CL-T7PAL-ER/K-PDZ-T7FNSI-T7CHS-T7CHI), pYH38(pACYCDuet1-T7matC-T7matB-T7ACS-T7FabF) and pET28a-T7 ygaP were transformed into BL21(DE3) to obtain the engineered strain J-2, used for chrysin fermentation with phenylalanine as a precursor.

The method for fermentation is as follows: The cells were cultured in LB solid medium (containing 80 µg/mL spectinomycin, 34 µg/mL chloramphenicol and 50 µg/mL kanamycin) overnight at 37°C. Single colony was picked and transferred to a 2mL LB liquid medium (containing 80 µg/mL spectinomycin, 34 µg/mL chloramphenicol and 50 µg/mL kanamycin) and incubated overnight. The bacterial fluid was transferred to a new 10mL MOPS liquid medium with antibiotics and incubated at 37°C and 250 r/min until OD600 reached 0.5-0.6. The culture was cooled down to 16°C, in a water bath, then IPTG inducer was added at a final concentration of 0.5mM, and sterile phenylalanine was added to a final concentration of 500 mg/L. The mixture was placed at 22°C for low-temperature induction and cultured for 72h at 250 r/min on the shaker. Samples were taken to measure the yield of chrysin.

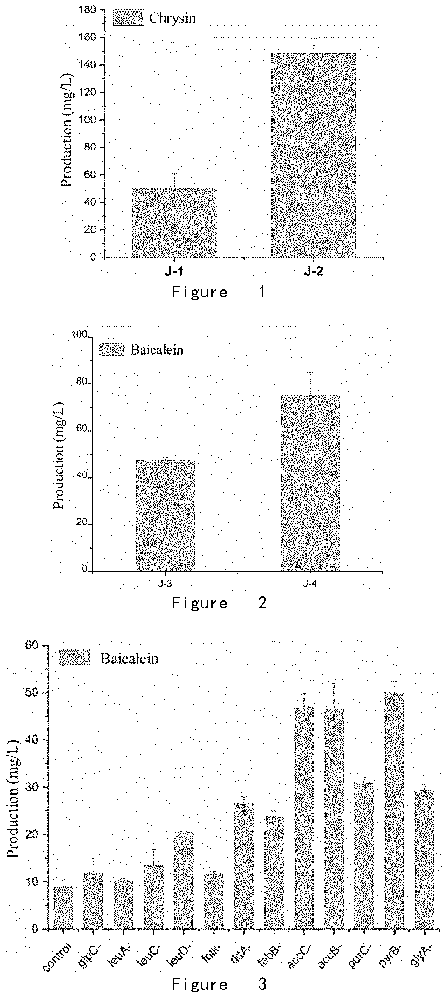

The results showed that the chrysin yield of the strain J-2 with ygaP overexpression was 2.99 times higher than that of strain J-1 (Figure 1).

### Example 2. Baicalein production by fermentation of engineered bacteria: over-expressed ygaP

### 1. Strain (J-3)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a were transformed into BL21(DE3) to obtain the engineered strain J-3, used for baicalein fermentation with phenylalanine as a precursor.

### 2. Strain with ygaP overexpression (J-4)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-T7 ygaP were transformed into BL21(DE3) to obtain the engineered strain J-4, used for baicalein fermentation with phenylalanine as a precursor.

The above two strains were used for fermentation and the fermentation method is as follows: The cells were cultured in LB solid medium (containing 80 µg/mL spectinomycin, 34 µg/mL chloramphenicol and 50 µg/mL kanamycin) overnight at 37°C. Single colony was picked and transferred to a 2mL LB liquid medium (containing 80 µg/mL spectinomycin, 34 µg/mL chloramphenicol and 50 µg/mL kanamycin) and incubated overnight. The bacterial fluid was transferred to a new 10mL MOPS liquid medium with antibiotics and incubated at 37°C and 250 r/min until OD600 reached 0.5-0.6. The culture was cooled down to 16°C, in a water bath, then IPTG inducer was added at a final concentration of 0.5mM, and sterile phenylalanine was added to a final concentration of 500 mg/L. The mixture was placed at 22°C for low-temperature induction and cultured for 72h at 250 r/min on the shaker. Samples were taken to measure the yield of baicalein.

The results showed that the baicalein yield of the strain J-4 with ygaP overexpression was 1.59 times higher than that of strain J-3 (Figure 2).

### Example 3. Regulation of baicalein permentation: Interfering with the expression of target genes

### 1. Control strain

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA were transformed into BL21(DE3) to obtain control strain, used for baicalein fermentation with phenylalanine as a precursor (the sRNA sequence in pET28a-sRNA has no target, only the sRNA hairpin structure exists).

### 2. Strain with glpC inhibition (glpC-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-glpC were transformed into BL21(DE3) to obtain the engineered strain glpC-, used for baicalein fermentation with phenylalanine as a precursor.

### 3. Strain with leuA inhibition (leuA-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-leuA were transformed into BL21(DE3) to obtain the engineered strain leuA-, used for baicalein fermentation with phenylalanine as a precursor.

### 4. Strain with leuC inhibition (leuC-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-leuC were transformed into BL21(DE3) to obtain the engineered strain leuC-, used for baicalein fermentation with phenylalanine as a precursor.

### 5. Strain with leuD inhibition (leuD-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-leuD were transformed into BL21(DE3) to obtain the engineered strain leuD-, used for baicalein fermentation with phenylalanine as a precursor.

### 6. Inhibition of folK strain (folK-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-folK were transformed into BL21(DE3) to obtain the engineered strain folK-, used for baicalein fermentation with phenylalanine as a precursor.

### 7. Strain with tktA inhibition (tktA-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-tkt were transformed into BL21(DE3) to obtain the engineered strain tktA-, used for baicalein fermentation with phenylalanine as a precursor.

### 8. Strain with folK inhibition (folK-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-fabB were transformed into BL21(DE3) to obtain the engineered strain fabB-, used for baicalein fermentation with phenylalanine as a precursor.

### 9. Strain with accC inhibition (accC-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-accC were transformed into BL21(DE3) to obtain the engineered strain accC-, used for baicalein fermentation with phenylalanine as a precursor.

### 10. Strain with accB inhibition (accB-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-accB were transformed into BL21(DE3) to obtain the engineered strain accB-, used for baicalein fermentation with phenylalanine as a precursor.

### 11. Strain with purC inhibition (purC-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-purC were transformed into BL21(DE3) to obtain the engineered strain purC-, used for baicalein fermentation with phenylalanine as a precursor.

### 12. Strain with pyrB inhibition (pyrB-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-pyrB were transformed into BL21(DE3) to obtain the engineered strain pyrB-, used for baicalein fermentation with phenylalanine as a precursor.

### 13. Inhibition of glyA strain (glyA-)

The plasmids pZZ42(pCDFDuet1-T7PDZlig-4CL-T7PAL-ER/K-PDZ-T7FNSI-T7CHS-T7CHI-T72B1trF6H-T7CPR), pYH38(pACYCDuet1-T7matC-T7matB-T7ACS-T7FabF) and pET28a-sRNA-glyA were transformed into BL21(DE3) to obtain the engineered strain glyA-, used for baicalein fermentation with phenylalanine as a precursor.

The above two strains were used for fermentation and the fermentation method is as follows: The cells were cultured in LB solid medium (containing 80 µg/mL spectinomycin, 34 µg/mL chloramphenicol and 50 µg/mL kanamycin) overnight at 37°C. Single colony was picked and transferred to a 2mL LB liquid medium (containing 80 µg/mL spectinomycin, 34 µg/mL chloramphenicol and 50 µg/mL kanamycin) and incubated overnight. The bacterial fluid was transferred to a new 10mL MOPS liquid medium with antibiotics and incubated at 37°C and 250 r/min until OD600 reached 0.5-0.6. The culture was cooled down to 16°C, in a water bath, then IPTG inducer was added at a final concentration of 0.5mM, and sterile phenylalanine was added to a final concentration of 500 mg/L. The mixture was placed at 22°C for low-temperature induction and cultured for 72h at 250 r/min on the shaker. Samples were taken to measure the yield of baicalein.

The results showed that compared to the control strain, the yield of baicalein increased as follows: 1.34 times in strain with glpC inhibition (glpC-), 1.16 times in strain with leuA inhibition (leuA-), 1.53 times in strain with leuC inhibition (leuC-), 2.32 times in strain with leuD inhibition (leuD-), 1.3 times in strain with folK inhibition (folK-), 3 times in strain with tktA inhibition (tktA-), 2.69 times in strain with fabB inhibition (fabB-), 5.31 times in strain with accC inhibition (accC-), 5.26 times in strain with accB inhibition (accB-), 3.51 times in strain with purC inhibition (purC-), 5.66 times in strain with pyrB inhibition (pyrB-) and 3.32 times in strain with glyA inhibition (glyA-). (Figure 3)

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A method for synthesizing a flavonoid compound, wherein the method comprises:
(1) providing a recombinant prokaryotic cell, comprising exogenous genes encoding the following enzymes: phenylalanine ammonia lyase, 4-coumaryl-CoA ligase, chalcone synthase, chalcone isomerase and flavone synthase I;
and, in the prokaryotic cell, the transmembrane protein rhodanese Ygap of *Escherichia coli* is up-regulated or a target gene or target gene combination selected from the following group is down-regulated: pyrB, accC, accB, purC, glyA, tktA, fabB, leuD, leuC, glpC, folk, leuA;
(2) using the formula (I) as a substrate to synthesize flavonoids with the prokaryotic cells of claim 1, wherein the flavonoid is chrysin compound; 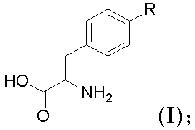 wherein, R comprises H or OH.

2. The method according to claim 1, wherein, in (1), the prokaryotic cell also comprises exogenous genes encoding the following enzymes: flavone 6-hydroxylase and cytochrome P450 oxidoreductase;
in (2), the flavonoid compound is baicalein compound.

3. The method according to claim 1 or 2, wherein, the up-regulation of transmembrane protein rhodanese Ygap of *Escherichia coli* comprises: introducing an exogenous gene encoding transmembrane protein rhodanese Ygap of *Escherichia coli* into the prokaryotic cell; preferably the exogenous gene encoding transmembrane protein rhodanese Ygap of *Escherichia coli* is introduced into the prokaryotic cell via an expression vector; or
the down-regulation of target gene comprises: knocking out or silencing the target gene in the cell, or inhibiting the activity of the target gene; preferably, knocking out or silencing the target gene in the cell comprises: silencing the target gene with a specific interfering molecule, knocking out the target gene by gene editing with a CRISPR system, knocking out the target gene by homologous recombination, or mutating the target gene by loss-of-function mutation; preferably, the interfering molecule comprises sRNA.

4. The method according to claim 3, wherein, the interfering molecule is sRNA, with the sRNA sequence targeting glpC is as shown in SEQ ID NO: 2;
the sRNA sequence targeting leuA is as shown in SEQ ID NO: 3;the sRNA sequence targeting leuC is as shown in SEQ ID NO: 4;
the sRNA sequence targeting leuD is as shown in SEQ ID NO: 5;
the sRNA sequence targeting folK is as shown in SEQ ID NO: 6;
the sRNA sequence targeting tktA is as shown in SEQ ID NO: 7;
the sRNA sequence targeting fabB is as shown in SEQ ID NO: 8;
the sRNA sequence targeting accC is as shown in SEQ ID NO: 9;
the sRNA sequence targeting accB is as shown in SEQ ID NO: 10;
the sRNA sequence targeting purC is as shown in SEQ ID NO: 11;
the sRNA sequence targeting pyrB is as shown in SEQ ID NO: 12;
the sRNA sequence targeting glyA is as shown in SEQ ID NO: 13.

5. The method according to claim 1 or 2, wherein, phenylalanine ammonia lyase and 4-coumaryl-CoA ligase are configured to form a complex;
preferably, the phenylalanine ammonia lyase and the 4-coumaryl-CoA ligase are approached to obtain a complex through the binding of the protein-protein interaction domain and the binding with its ligand, or the phenylalanine ammonia lyase and the 4-coumaryl-CoA ligase are linked directly or using a linker to obtain a fused protein complex;
preferably, the protein-protein interaction domain comprises PDZ domain and the ligand thereof is PDZ ligand; the phenylalanine ammonia lyase and the 4-coumaryl-CoA ligase are separately fused to PDZ and PDZ ligand; preferably, the phenylalanine ammonia lyase is fused to PDZ and the 4-coumaryl-CoA ligase is fused to PDZ ligand.

6. The method according to claim 1 or 2, wherein, in (1), the prokaryotic cell also comprises exogenous genes encoding enzymes promoting malonyl-CoA production; preferably comprising matC, matB, ACS, FabF.

7. The method according to claim 1 or 2, wherein, the prokaryotic cell is a cell with a substrate synthesis pathway of formula (I); preferably, the prokaryotic cell is an *Escherichia coli* cell.

8. A prokaryotic cell for synthesizing flavonoid compound, wherein, the prokaryotic cell comprises exogenous genes encoding the following enzymes: phenylalanine ammonia lyase, 4-coumaryl-CoA ligase, chalcone synthase, chalcone isomerase and flavone synthase I;
and, in the prokaryotic cell, the transmembrane protein rhodanese Ygap of *Escherichia coli* is up-regulated or a target gene or target gene combination selected from the following groups is down-regulated: pyrB, accC, accB, purC, glyA, tktA, fabB, leuD, leuC, glpC, folk, leuA;
the flavonoid compound is chrysin compound.

9. The prokaryotic cell according to claim 8, wherein the prokaryotic cell also comprises exogenous genes encoding the following enzymes: flavone 6-hydroxylase and cytochrome P450 oxidoreductase; the flavonoid compound is baicalein compound.

10. A use of the prokaryotic cell according to claim 8 or 9 for synthesizing a flavonoid compound; preferably, the flavonoid compound comprises: chrysin compound, baicalein compound.

11. A kit for producing a flavonoid compound, wherein the kit comprises the prokaryotic cell according to claim 8 or 9.

12. A kit for constructing host cells for synthesizing flavonoid compounds, wherein the kit comprises:
(a) a construct expressing phenylalanine ammonia lyase, 4-coumaryl-CoA ligase, chalcone synthase, chalcone isomerase and flavone synthase I;
(b) a construct expressing transmembrane protein rhodanese Ygap of *Escherichia coli;* or a construct expressing a down-regulator, wherein the down-regulator down-regulates the target gene or target gene combination selected from the following group: pyrB, accC, accB, purC, glyA, tktA, fabB, leuD, leuC, glpC, folk, leuA;
optionally, the kit further comprises a construct expressing genes encoding flavone 6-hydroxylase and cytochrome P450 oxidoreductase.

13. A use of regulators for promoting biosynthesis of flavonoid compounds, the regulators are selected from:
(i) transmembrane protein rhodanese Ygap of *Escherichia coli* or a up-regulator thereof; or
(ii) a down-regulator, wherein the down-regulator down-regulates the target gene or target gene combination selected from the following group: pyrB, accC, accB, purC, glyA, tktA, fabB, leuD, leuC, glpC, folk, leuA.
